(19) Europäisches Patentamt

European Patent Office

Office européen des brevets

(11) **EP 3 339 838 A1**

(12) **EUROPEAN PATENT APPLICATION**
published in accordance with Art. 153(4) EPC

(43) Date of publication:
**27.06.2018 Bulletin 2018/26**

(21) Application number: **16839153.0**

(22) Date of filing: **17.08.2016**

(51) Int Cl.:
***G01N 21/35*** (2014.01)

(86) International application number:
**PCT/JP2016/073971**

(87) International publication number:
**WO 2017/033806 (02.03.2017 Gazette 2017/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **21.08.2015 JP 2015163854**

(71) Applicant: **Daikin Industries, Ltd.**
**Osaka 530-8323 (JP)**

(72) Inventors:
• **TAKAHASHI, Kanako**
**Kita-ku**
**Osaka-shi**
**Osaka 530-8323 (JP)**

• **YAMAMOTO, Ikuo**
**Kita-ku**
**Osaka-shi**
**Osaka 530-8323 (JP)**
• **HASEGAWA, Takeshi**
**Sakyo-ku, Kyoto-shi**
**Kyoto 606-8501 (JP)**
• **SHIMOAKA, Takafumi**
**Sakyo-ku, Kyoto-shi**
**Kyoto 606-8501 (JP)**

(74) Representative: **Hoffmann Eitle**
**Patent- und Rechtsanwälte PartmbB**
**Arabellastraße 30**
**81925 München (DE)**

(54) **ANALYSIS OF FLUORINE-CONTAINING POLYMER BY USING INFRARED SPECTROSCOPY**

(57)     Disclosed is a method for analyzing an item containing a fluorine-containing surface treatment agent by means of an infrared attenuated total reflection method (IR-ATR method) using a Fourier transformation infrared spectrometer (FT-IR) with which the number of carbon atoms in a fluoroalkyl group included in the fluorine-containing surface treatment agent is obtained, wherein the fluorine-containing surface treatment agent contains a fluorine-containing polymer as an active ingredient, and the fluorine-containing polymer has a repeat unit derived from a fluorine-containing monomer represented by a formula: $CH_2=C(-X)-C(=O)-Y-Z-Rf$ (in the formula, X represents a hydrogen atom, a monovalent organic group, or a halogen atom, Y represents -O- or -NH-, Z represents a direct bond or a divalent organic group, and Rf represents a fluoroalkyl group having 1-20 carbon atoms). Further provided is a method for easily analyzing the number of carbon atoms in a fluoroalkyl group in an item containing a fluorine-containing surface treatment agent in which a fluorine-containing polymer is an active ingredient.

EP 3 339 838 A1

Fig. 1

## Description

### TECHNICAL FIELD

The present invention relates to an analytical method of determining the number of carbon atoms in a fluoroalkyl group of a fluorine-containing surface treatment agent comprising a fluorine-containing polymer as an active ingredient by an infrared attenuated total reflection method (IR-ATR method).

### BACKGROUND ART

A method of measuring a surface segregation property of a fluorine-containing hydrocarbon chain in a fluorine-containing organic compound has been proposed.

A technique of locally analyzing a sample by XPS or a method using X-ray absorption from orbital radiation is disclosed (e.g., K. Honda, M. Morita, H. Otsuka, A. Takahara, Macromolecules 38 (13), 5699-5705 (2005), and K. Honda, M. Morita, O. Sakata, S. Sasaki and A. Takahara, Macromolecules, 43 (1), 454-460 (2010)).

However, a large scale of an apparatus and a severe restriction on machine time give a large technical barrier to analysis of a fabric sample at hand.

WO 2015/020100 discloses a method of collecting a small piece of a processed product of, for example, fiber, clothing and paper subjected to water- and oil-repellent finishing and analyzing a volatile component obtained by heating the piece by gas chromatography to measure the number of carbon atoms in a fluorine-containing organic compound. However, this method comprises heating a sample and therefore may cause damage to the sample.

Takeshi Hasegawa et al., "Stratified Dipole-Arrays Model Accounting for Bulk Properties Specific to Perfluoroalkyl Compounds", ChemPlusChem, 79, 1421-1425 (2014) discloses a method of analyzing a perfluoroalkyl compound by the IR-ATR method to measure the number of carbon atoms in a perfluoroalkyl group. However, the perfluoroalkyl compound is a small molecule of $CF_3(CF_2)_n-(CH_2)_{12-n}-COOH(n = 3, 5, 7, 9)$ in which a perfluoroalkyl group is introduced into myristic acid. Furthermore, the measurement is performed on a sample of a monomolecular film formed on a gold substrate. Additionally, this spectrum corresponds to the measurement of only the Longitudinal optic energy loss function (LO function) spectrum.

Also in Takeshi Hasegawa et al., "An Origin of Complicated Infrared Spectra of Perfluoroalkyl Compounds Involving a Normal Alkyl Group", Chemistry Letters, 44, 834-836 (2015) and Takeshi Hasegawa, "Understanding of the intrinsic difference between normal- and perfluoro-alkyl compounds toward total understanding of material properties", Chemical Physics Letters, 627, 64-66 (2015), a method of analyzing a perfluoroalkyl compound is disclosed.

### PRIOR ART DOCUMENTS

### PATENT DOCUMENTS

Patent Document 1: WO 2015/020100

### NON-PATENT LITERATURE

Nonpatent Literature 1: K. Honda et al. Macromolecules 38 (13), 5699-5705 (2005).
Nonpatent Literature 2: K. Honda et al. Macromolecules, 43 (1), 454-460 (2010).
Nonpatent Literature 3: Takeshi Hasegawa et al., "Stratified Dipole-Arrays Model Accounting for Bulk Properties Specific to Perfluoroalkyl Compounds", ChemPlusChem, 79, 1421-1425 (2014).
Nonpatent Literature 4: T. Hasegawa et al., "An Origin of Complicated Infrared Spectra of Perfluoroalkyl Compounds Involving a Normal Alkyl Group", Chemistry Letters, 44, 834-836 (2015).
Nonpatent Literature 5: T. Hasegawa, "Understanding of the intrinsic difference between normal- and perfluoro-alkyl compounds toward total understanding of material properties", Chemical Physics Letters, 627, 64-66 (2015).

### SUMMARY OF THE INVENTION

### PROBLEM TO BE SOLVED BY THE INVENTION

The present invention provides an analytical method of conveniently determining the number of carbon atoms in a fluoroalkyl group (preferably the number of carbon atoms in a perfluoroalkyl group) in an article comprising a fluorine-containing surface treatment agent comprising a fluorine-containing polymer as an active ingredient.

## MEANS FOR SOLVING PROBLEM

The present invention provides a method of analyzing an article comprising a fluorine-containing surface treatment agent to determine the number of carbon atoms in a fluoroalkyl group of the fluorine-containing surface treatment agent by an infrared attenuated total reflection method (IR-ATR method), wherein
the fluorine-containing surface treatment agent comprises a fluorine-containing polymer as an active ingredient, and
the fluorine-containing polymer has a repeating unit derived from a fluorine-containing monomer represented by the formula:

$$CH_2=C(-X)-C(=O)-Y-Z-Rf$$

where X is a hydrogen atom, a monovalent organic group, or a halogen atom,
Y is -O- or -NH-,
Z is a direct bond or a divalent organic group, and
Rf is a fluoroalkyl group having 1 to 20 carbon atoms.

## EFFECT OF THE INVENTION

The Rf group in the fluorine-containing polymer is the active ingredient of the fluorine-containing surface treatment agent and has the property, which is not found in a hydrocarbon chain, that a wavenumber position of a $CF_2$ symmetric stretching vibration band largely depends on the Rf chain length. A difference of the Rf chain length (preferably between Cg and $C_6$) can be distinguished by infrared spectroscopy which is a simple method of nondestructive and room-temperature measurement. By multivariate analysis of a spectrum obtained by incorporating an incident-angle variable ATR apparatus into a Fourier-transform infrared spectrometer (FT-IR), a spectrum of only a coating layer can be extracted, so that blank data (e.g., data of a blank fabric) is not necessary.

## BRIEF DESCRIPTION OF DRAWINGS

Fig. 1 is bulk IR-ATR spectra obtained in Examples 1 to 5.
Fig. 2 is an IR-ATR spectrum that is a difference spectrum between polymer-treated nylon fabric and untreated nylon fabric obtained in Examples 6 and 7.
Fig. 3 is an IR-ATR spectrum that is a difference spectrum between polymer-treated polyester fabric and untreated nylon fabric obtained in Examples 8 and 9.
Fig. 4 is a result of extraction of a spectrum of only a coating layer by multivariate analysis of an IR-ATR spectrum of polymer-treated nylon fabric obtained in Examples 10 and 11.

## MODES FOR CARRYING OUT THE INVENTION

In the present invention, the article comprising the fluorine-containing surface treatment agent is analyzed by using the infrared attenuated total reflection method (IR-ATR method).
The "article comprising a fluorine-containing surface treatment agent" is generally an article treated (surface-treated) with the fluorine-containing surface treatment agent. By treating the article with the fluorine-containing surface treatment agent, a coating layer made of the fluorine-containing surface treatment agent is formed on the article. The fluorine-containing surface treatment agent is generally a fluorine-containing water- and oil-repellent agent and/or a fluorine-containing soil-resistant agent. Examples of the "article", particularly, an object (substrate) to be treated with the fluorine-containing surface treatment agent (e.g., the fluorine-containing water- and oil-repellent agent) include textiles (e.g., yarn, knitted fabric, woven fabric, nonwoven fabric, and clothing, bedding, curtains, rugs made thereof), stone materials, filters (e.g., electrostatic filters), dust protective masks, parts of fuel cells (e.g., gas diffusion electrodes and gas diffusion supports), glass, paper, wood, leather, fur, asbestos, brick, cement, metal and oxide, ceramic products, plastic, painted surfaces, and plaster. Particularly, the textile may be a carpet. The "treatment" means applying a treatment agent to the object to be treated (substrate) by, for example, dipping, spraying or coating. The treatment gives the result that a fluorine-containing polymer which is an active component of the treatment agent is adhered to surfaces of the substrate and/or penetrated into internal parts of the substrate.

The fluorine-containing surface treatment agent generally comprises the fluorine-containing polymer as an active ingredient. The fluorine-containing polymer generally has a repeating unit derived from a fluorine-containing monomer represented by the formula:

$$CH_2=C(-X)-C(=O)-Y-Z-Rf$$

where X is a hydrogen atom, a monovalent organic group, or a halogen atom,
Y is -O- or -NH-,
Z is a direct bond or a divalent organic group, and
Rf is a fluoroalkyl group having 1 to 20 carbon atoms.

Y is preferably -O-.
Specific examples of Z are:

a direct bond,
a linear alkylene group or a branched alkylene group having 1 to 20 carbon atoms [e.g., a group represented by the formula $-(CH_2)_x-$ wherein x is 1 to 10],
or
a group represented by the formula $-SO_2N(R^1)R^2-$ or the formula $-CON(R^1)R^2$ wherein $R^1$ is an alkyl group having 1 to 10 carbon atoms and $R^2$ is a linear alkylene group or branched alkylene group having 1 to 10 carbon atoms,
or
a group represented by the formula $-CH_2CH(OR^3)CH_2-$ wherein $R^3$ represents a hydrogen atom, or an acyl group having 1 to 10 carbon atoms (e.g., formyl or acetyl),
or
a group represented by the formula $-Ar-CH_2-$ wherein Ar is an arylene group having a substituent as necessary,
or
a $-(CH_2)_m-SO_2-(CH_2)_n-$ group or a $-(CH_2)_m-S-(CH_2)_n-$ group wherein m is 1 to 10 and n is 0 to 10.

In the present invention, Z is preferably a direct bond, an alkylene group having 1 to 20 carbon atoms, or $-SO_2N(R^1)R^2-$.
Although Rf is a fluoroalkyl group which may have a hydrogen atom, Rf is preferably a perfluoroalkyl group free of a hydrogen atom. Specific examples of Rf are $-CF_3$, $-C_2F_5$, $-C_3F_7$, $-C_4F_9$, $-C_5F_{11}$, $-C_6F_{13}$, $-C_8F_{17}$, $-C_{10}F_{21}$, and $-C_{12}F_{25}$.
The fluorine-containing polymer may be a homopolymer or a copolymer of the fluorine-containing monomer. The fluorine-containing polymer may have a repeating unit derived from a non-fluorine monomer (e.g., a (meth)acrylate ester free of a fluorine atom) in addition to the repeating unit derived from the fluorine-containing monomer.
A spectrum (raw spectrum) of a bulk sample is obtained by the infrared attenuated total reflection method (IR-ATR method) using FT-IR. From the raw spectrum, an $\alpha$ spectrum ($\alpha=4\pi n''/\lambda$ ($\lambda$: wavelength)) is obtained by using Kramers-Kronig relations. The $\alpha$ spectrum is further subjected to Kramers-Kronig transformation to obtain a refractive index n'. The complex refractive index n of the sample is expressed as n = n' + in'' as the sum of a real part n' and an imaginary part n''. A complex permittivity function is then obtained by using a relational expression $\varepsilon=n^2$ (the complex permittivity of the sample:$\varepsilon$), and two energy loss functions (TO and LO functions) are obtained therefrom.

TO: Im($\varepsilon$)
LO: Im ($-1/\varepsilon$)

The TO and LO functions closely correspond to infrared transmission (Tr) and reflection absorption (RA) spectra of a thin film, which makes it possible to compare the spectrum of the bulk sample with a spectrum of a thin film sample.
A sample (article) is preferably analyzed by using the $\alpha$ spectrum or the TO function. A combination of the TO function and the LO function may be used.
Analysis of the spectrum of the IR-ATR method can be referred to, for example, V.P. Tolstoy, I.V. Chemyshowa, V.A. Skryshevsky, Handbook of Infrared Spectroscopy of Ultrathin Films, Wiley, Chichester, (2003) (the disclosure of which is incorporated herein by reference).
The Kramers-Kronig relations (and the Kramers-Kronig transformation) are expressed by, for example,

$$\varepsilon'(\omega) - \varepsilon_\infty = \frac{2}{\pi} P \int_0^\infty \frac{\varepsilon''(\omega')\omega'}{\omega'^2 - \omega^2} d\omega'$$

$$\varepsilon''(\omega) = \frac{2\omega}{\pi} P \int_0^\infty \frac{\varepsilon'(\omega') - \varepsilon_\infty}{\omega'^2 - \omega^2} d\omega'$$

[$\varepsilon$: permittivity, $\varepsilon_\infty$: high-frequency permittivity, $\omega$: angular speed, P∫: Cauchy principal value integration]

or

$$\varphi(v_g) = \frac{2v_g}{\pi} \int_0^\infty \frac{ln\sqrt{R(v)}}{v^2 - v_g^2} dv$$

[$\varphi$: phase change, $v$: wavenumber, R: reflection spectrum].

The measurement result of the IR-ATR method is the superposition of the TO and LO functions. The TO and LO function spectra can be compared with Tr and RA spectra having corresponding absolute values and relative intensities at peak positions.

The ATR method is preferably an incident-angle variable ATR method or an incident-angle variable polarization ATR method. By using the incident-angle variable polarization ATR method, the spectrum of only the coating layer can be extracted from the sample having the coating layer disposed on the substrate.

A thin film sample adsorbed on an infrared-transparent substrate having a smooth surface can use a multiple-angle incidence resolution spectrometry (MAIRS method) which is capable of obtaining an orientation structure such as an orientation angle of a chemical bond at an interface. The MAIRS method (including a p-MAIRS) is a measurement technique with which an IP spectrum (in-plane spectrum) and an OP spectrum (out-of-plane spectrum) respectively corresponding to the conventional infrared transmission (Tr) and reflection absorption (RA) spectra can be obtained from one sample at the same time.

The TO and LO functions are obtained also by the MAIRS method, and TO corresponds to the IP spectrum [in-plane transmission] (MAIRS IP) while LO corresponds to the OP spectrum [out-of-plane reflection absorption] (MAIRS OP).

It was found that the fluorine-containing polymer has a large difference between the TO and LO spectra in comparison with $CF_3(CF_2)_n$-$(CH_2)_{12-n}$-COOH (n=3, 5, 7, 9) in which a perfluoroalkyl group is introduced into myristic acid.

Particularly, a fluorine-containing polymer having a repeating unit derived from a fluorine-containing monomer containing the perfluoroalkyl group having 4 carbon atoms (preferably $CF_3CF_2CF_2CF_2$-$CH_2CH_2OCOCH=CH_2$ (C4SFA)) has a large difference in peak position of the $CF_2$ symmetric stretching vibration band appearing in the TO and LO spectra. Additionally, since C4SFA is a gel so that the structural anisotropy of the sample can be ignored, and is excellent in reproducibility of sample fabrication, the fluorine-containing monomer containing the perfluoroalkyl group having 4 carbon atoms can be used as a standard sample of the infrared MAIRS method.

In the infrared MAIRS method (including the p-MAIRS method), the IP spectrum (in-plane spectrum) and the OP spectrum (out-of-plane spectrum) corresponding to the conventional infrared transmission (Tr) and reflection absorption (RA) spectra can be obtained at the same time from one thin film sample fabricated on a smooth substrate. In order to confirm whether MAIRS measurement is correctly performed, a band "intensity ratio" reflecting the orientation appearing in the IP and OP spectra has been conventionally utilized by using an oriented sample. However, for the oriented sample, a thin film having a known structure such as an LB film and a SAM film must be fabricated as a standard sample, so that the standard sample fabrication is a high barrier for both of measurers and spectrometer manufacturers.

This may be overcome by using a confirmation method of checking a difference in the band "position" of the IP and OP spectra, instead of the band intensity ratio. This utilizes the fact that the TO and LO energy loss function spectra respectively appear in the IP and OP spectra even in the same thin film sample, and band positions of strong absorption intensity appear at different positions (TO-LO splitting). C4SFA has a distinctly large TO-LO splitting of 4.5 cm$^{-1}$, which can be easily measured. Additionally, the $CF_2$ symmetric stretching vibration band having a highest utility value is in the vicinity of 1150 cm$^{-1}$, does not overlap with a band of water vapor, and therefore can be measured regardless of a disposition condition of a spectrometer, which is ideal for the standard sample.

In the present invention, the IR-ATR measurement of a blank sample (i.e., an article free of a surface treatment) may not be performed. The spectrum of the coating layer made of the fluorine-containing surface treatment agent can be obtained (e.g., by the incident-angle variable ATR method) even without taking a difference spectrum between the blank sample and a surface-treated article, so that an article comprising the fluorine-containing surface treatment agent can be analyzed.

**EXAMPLES**

Infrared total reflection absorption measurement was performed by using the following apparatus.

(1) Examples 1 to 9

Apparatus: Nicolet 6700 (manufactured by ThermoFisher Scientific) FT-IR
Incident angle: 45 degrees
Number of reflections: once
Prism: Ge

(2) Examples 10 and 11

Incident-angle variable ATR apparatus: variable angle reflection/ATR Seagull (manufactured by Harrik Scientific)
Number of reflections: once
Prism: Ge

Synthesis Example 1

After 30.0 g of $C_8F_{17}CH_2CH_2OCOCH=CH_2/C_{10}F_{21}CH_2CH_2OCOC(CH_3)=CH_2$ (weight ratio: 80/20) and 81.0 g of butyl acetate were stirred for dissolution in a four-neck flask and maintained at 65°C with nitrogen purge, 1.3 g of an organic peroxide was added and reacted at 65°C for 8 hours to obtain a solution of a polymer. The conversion rate of monomer determined by gas chromatograph was 95 % or more. Butyl acetate was removed from the resulting polymerization liquid by an evaporator to obtain the polymer.

Synthesis Example 2

After 30.0 g of $CF_3CF_2-(CF_2CF_2)_2-CH_2CH_2OCOC(Cl)=CH_2$ and 81.0 g of butyl acetate were stirred for dissolution in a four-neck flask and maintained at 65°C with nitrogen purge, 1.3 g of an organic peroxide was added and reacted at 65°C for 8 hours to obtain a solution of a polymer. The conversion rate of monomer determined by gas chromatograph was 95 % or more. Butyl acetate was removed from the resulting polymerization liquid by an evaporator to obtain the polymer.

Synthesis Example 3

After 30.0 g of $CF_3CF_2-(CF_2CF_2)_2-CH_2CH_2OCOC(CH_3)=CH_2$ and 81.0 g of butyl acetate were stirred for dissolution in a four-neck flask and maintained at 65°C with nitrogen purge, 1.3 g of an organic peroxide was added and reacted at 65°C for 8 hours to obtain a solution of a polymer. The conversion rate of monomer determined by gas chromatograph was 95 % or more. Butyl acetate was removed from the resulting polymerization liquid by an evaporator to obtain the polymer.

Synthesis Example 4

After 30.0 g of $CF_3CF_2-(CF_2CF_2)_2-CH_2CH_2OCOCH=CH_2$ and 81.0 g of butyl acetate were stirred for dissolution in a four-neck flask and maintained at 65°C with nitrogen purge, 1.3 g of an organic peroxide was added and reacted at 65°C for 8 hours to obtain a solution of a polymer. The conversion rate of monomer determined by gas chromatograph was 95 % or more. Butyl acetate was removed from the resulting polymerization liquid by an evaporator to obtain the polymer.

Synthesis Example 5

After 30.0 g of $CF_3CF_2CF_2CF_2CH_2CH_2OCOCH=CH_2$ and 81.0 g of butyl acetate were stirred for dissolution in a four-neck flask and maintained at 65°C with nitrogen purge, 1.3 g of an organic peroxide was added and reacted at 65°C for 8 hours to obtain a solution of a polymer. The conversion rate of monomer determined by gas chromatograph was 95 % or more. Butyl acetate was removed from the resulting polymerization liquid by an evaporator to obtain the polymer.

Synthesis Example 6

In a 1 L four-neck flask, 204 g of $C_8F_{17}CH_2CH_2OCOC(CH_3)=CH_2/C_{10}F_{21}CH_2CH_2OCOC(CH_3)=CH_2$ (weight ratio: 80/20), 75.8 g of a water-soluble glycol-based solvent, 446 g of pure water, 20.2 g of polyoxyethylene alkyl ether, and 2.6 g of a cationic emulsifier were added, heated at 60°C, and then emulsified by a high-pressure homogenizer. Further, 1.9 g of an azo group-containing water-soluble initiator was added and reacted at 60°C for 3 hours to obtain an aqueous dispersion of a polymer. The conversion rate of monomer determined by gas chromatograph was 95 % or more. Pure water was added to adjust a solid content concentration to 30 wt%.

Synthesis Example 7

In a 1 L four-neck flask, 204 g of $C_6F_{13}CH_2CH_2OCOC(CH_3)=CH_2$, 75.8 g of a water-soluble glycol-based solvent, 446 g of pure water, 20.2 g of polyoxyethylene alkyl ether, and 2.6 g of a cationic emulsifier were added, heated at 60°C, and then emulsified by a high-pressure homogenizer. Further, 1.9 g of an azo group-containing water-soluble initiator was added and reacted at 60°C for 3 hours to obtain an aqueous dispersion of a polymer. The conversion rate of monomer determined by gas chromatograph was 95 % or more. Pure water was added to adjust a solid content concentration to 30 wt%.

Comparative Synthesis Example 1

In a 1 L four-neck flask, 179 g of stearyl acrylate, 75.8 g of a water-soluble glycol-based solvent, 446 g of pure water, 20.2 g of polyoxyethylene alkyl ether, and 2.6 g of a cationic emulsifier were added, heated at 60°C, and then emulsified by a high-pressure homogenizer. Further, 1.9 g of an azo group-containing water-soluble initiator was added and reacted at 60°C for 3 hours to obtain an aqueous dispersion of a polymer. The conversion rate of monomer determined by gas chromatograph was 95 % or more. Pure water was added to adjust a solid content concentration to 30 wt%.

Example 1

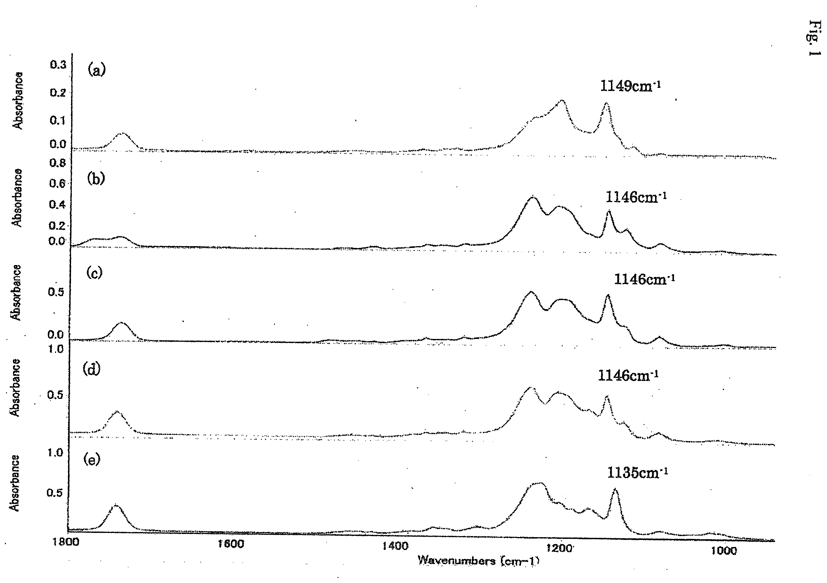

The polymer obtained by air drying of the polymerization liquid obtained in Synthesis Example 1 was placed on a prism of an infrared ATR apparatus and subjected to IR-ATR measurement. By converting the obtained data to an $\alpha$ spectrum, a $CF_2$ symmetric stretching vibration peak was observed at 1149 cm$^{-1}$. The peak at 1149 cm$^{-1}$ is characteristic of the $C_8F_{17}$ group. The IR spectrum is shown in Fig. 1(a).

Example 2

The fluorine-containing polymer obtained in Synthesis Example 2 was subjected to IR-ATR measurement in the same manner as in Example 1. By converting the obtained data to an $\alpha$ spectrum, a $CF_2$ symmetric stretching vibration peak was observed at 1146 cm$^{-1}$. The peak at 1146 cm$^{-1}$ is characteristic of the $C_6F_{13}$ group. The IR spectrum is shown in Fig. 1(b).

Example 3

The fluorine-containing polymer obtained in Synthesis Example 3 was subjected to IR-ATR measurement in the same manner as in Example 1. By converting the obtained data to an $\alpha$ spectrum, a $CF_2$ symmetric stretching vibration peak was observed at 1146 cm$^{-1}$. The peak at 1146 cm$^{-1}$ is characteristic of the $C_6F_{13}$ group. The IR spectrum is shown in Fig. 1(c).

Example 4

The fluorine-containing polymer obtained in Synthesis Example 4 was subjected to IR-ATR measurement in the same manner as in Example 1. By converting the obtained data to an $\alpha$ spectrum, a $CF_2$ symmetric stretching vibration peak was observed at 1146 cm$^{-1}$. The peak at 1146 cm$^{-1}$ is characteristic of the $C_6F_{13}$ group. The IR spectrum is shown in Fig. 1(d).

Example 5

The fluorine-containing polymer obtained in Synthesis Example 5 was subjected to IR-ATR measurement in the same manner as in Example 1. By converting the obtained data to an $\alpha$ spectrum, a $CF_2$ symmetric stretching vibration peak was observed at 1135 cm$^{-1}$. The peak at 1135 cm$^{-1}$ is characteristic of the $C_4F_9$ group. The IR spectrum is shown in Fig. 1(e).

Example 6

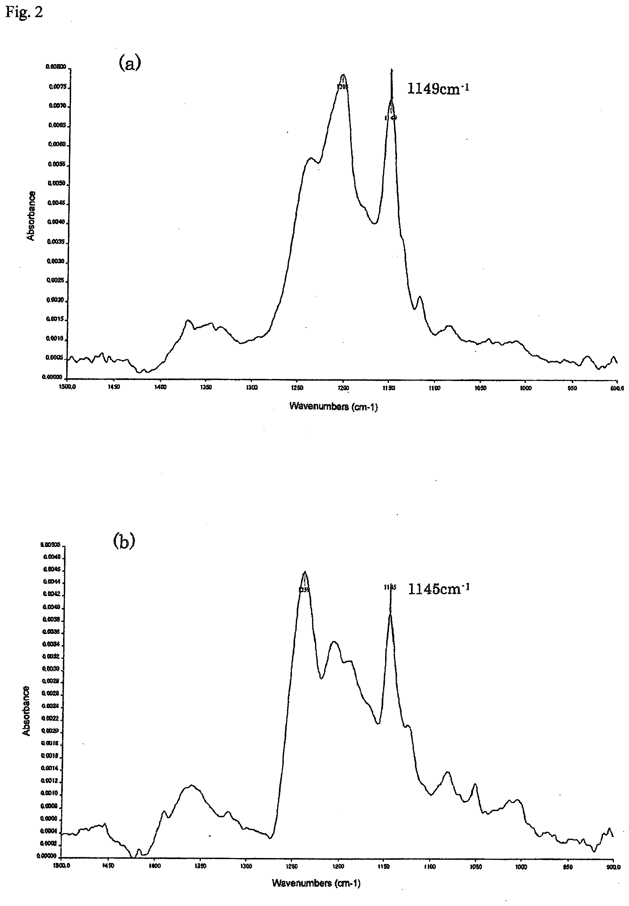

The aqueous dispersion of the polymer obtained in Synthesis Example 6 was diluted with water to 1.5 wt% to give a treatment liquid. A nylon cloth was immersed in the treatment liquid, squeezed with a mangle at 4 kg/cm$^2$ and 4 m/min, and heat-treated at 170°C for 1 minute to obtain a treated cloth. The treated cloth was placed on a prism of an infrared ATR apparatus and subjected to IR-ATR measurement. The obtained data was converted to an $\alpha$ spectrum. By taking a difference spectrum from the untreated nylon cloth, a $CF_2$ symmetric stretching vibration peak was observed at 1149 cm$^{-1}$. The peak at 1149 cm$^{-1}$ is characteristic of the $C_8F_{17}$ group. The IR spectrum is shown in Fig. 2(a).

Example 7

With using the aqueous dispersion of the polymer obtained in Synthesis Example 7, a treated cloth was obtained and subjected to IR-ATR measurement in the same manner as in Example 6. The obtained data was converted to an $\alpha$ spectrum. By taking a difference spectrum from the untreated nylon cloth, a $CF_2$ symmetric stretching vibration peak was observed at 1145 cm$^{-1}$. The peak at 1145 cm$^{-1}$ is characteristic of the $C_6F_{13}$ group. The IR spectrum is shown in Fig. 2(b).

Example 8

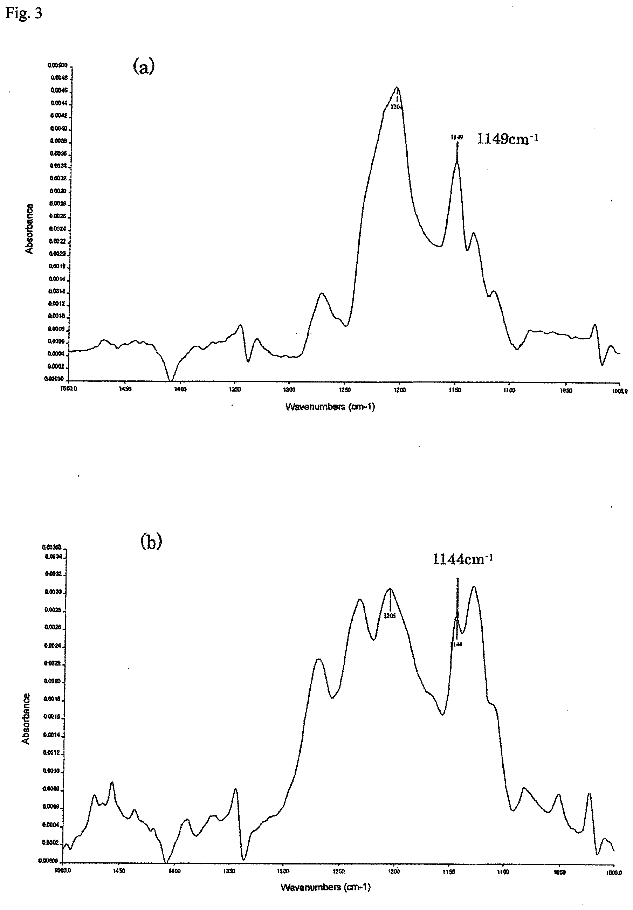

The aqueous dispersion of the polymer obtained in Synthesis Example 6 was diluted with water to 1.5 wt% to give a treatment liquid. A polyester cloth was immersed in the treatment liquid, squeezed with a mangle at 4 kg/cm$^2$ and 4 m/min, and heat-treated at 170°C for 1 minute to obtain a treated cloth. The treated cloth was placed on a prism of an infrared spectrometer and subjected to IR-ATR measurement. The obtained data was converted to an $\alpha$ spectrum. By taking a difference spectrum from the untreated polyester cloth, a $CF_2$ symmetric stretching vibration peak was observed at 1149 cm$^{-1}$. The peak at 1149 cm$^{-1}$ is characteristic of the $C_8F_{17}$ group. The IR spectrum is shown in Fig. 3(a).

Example 9

With using the aqueous dispersion of the polymer obtained in Synthesis Example 7, a treated cloth was obtained and subjected to IR-ATR measurement in the same manner as in Example 6. The obtained data was converted to an $\alpha$ spectrum. By taking a difference spectrum from the untreated polyester cloth, a $CF_2$ symmetric stretching vibration peak was observed at 1144 cm$^{-1}$. The peak at 1144 cm$^{-1}$ is characteristic of the $C_6F_{13}$ group. The IR spectrum is shown in Fig. 3(b).

Example 10

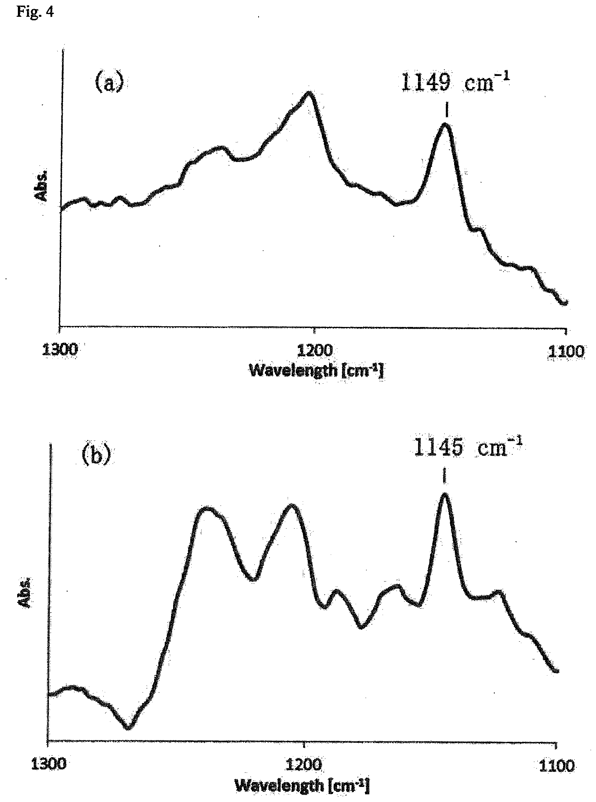

An incident-angle variable ATR apparatus was incorporated in a Fourier-transform infrared spectrometer (FT-IR), and the IR-ATR measurement was performed by using the treated fabric obtained in Example 6. The obtained data was converted to an $\alpha$ spectrum to extract the spectrum of only the coating layer by multivariate analysis. A $CF_2$ symmetric stretching vibration peak was observed at 1149 cm$^{-1}$. The peak at 1149 cm$^{-1}$ is characteristic of the $C_8F_{17}$ group. Even without performing IR-ATR measurement of untreated nylon cloth, the $CF_2$ symmetric stretching vibration peak was observed at the same position as in Example 6. The spectrum of the coating layer is shown in Fig. 4(a).

Example 11

An incident-angle variable ATR apparatus was incorporated in a Fourier-transform infrared spectrometer (FT-IR), and the IR-ATR measurement was performed by using the treated fabric obtained in Example 7. The obtained data was converted to an $\alpha$ spectrum to extract the spectrum of only the coating layer by multivariate analysis. A $CF_2$ symmetric stretching vibration peak was observed at 1145 cm$^{-1}$. The peak at 1145 cm$^{-1}$ is characteristic of the $C_6F_{13}$ group. Even without performing IR-ATR measurement of untreated nylon cloth, the $CF_2$ symmetric stretching vibration peak was observed at the same position as in Example 7. The spectrum of the coating layer is shown in Fig. 4(b).

Comparative Example 1

With using the aqueous dispersion of the polymer obtained in Comparative Synthesis Example 1, a treated cloth was obtained and subjected to IR-ATR measurement in the same manner as in Example 6. The obtained data was converted to an α spectrum. Although a difference spectrum from the untreated nylon cloth was taken, a $CF_2$ symmetric stretching vibration peak was not observed.

**INDUSTRIAL APPLICABILITY**

According to the method of the present invention, the number of carbon atoms in the fluoroalkyl group of the fluorine-containing surface treatment agent can be determined by the infrared attenuated total reflection method (IR-ATR method).

**Claims**

1. A method of analyzing an article comprising a fluorine-containing surface treatment agent to determine the number of carbon atoms in a fluoroalkyl group of the fluorine-containing surface treatment agent by an infrared attenuated total reflection method (IR-ATR method) using a Fourier transform infrared spectrometer (FT-IR), wherein
   the fluorine-containing surface treatment agent comprises a fluorine-containing polymer as an active ingredient, and the fluorine-containing polymer has a repeating unit derived from a fluorine-containing monomer represented by the formula:

   $$CH_2=C(-X)-C(=O)-Y-Z-Rf$$

   where X is a hydrogen atom, a monovalent organic group, or a halogen atom,
   Y is -O- or -NH-,
   Z is a direct bond or a divalent organic group, and
   Rf is a fluoroalkyl group having 1 to 20 carbon atoms.

2. The method according to claim 1, wherein the article is a film of the fluorine-containing surface treatment agent or the article has a layer of the fluorine-containing surface treatment agent positioned on a substrate selected from the group consisting of a textile, a stone material, a filter, a dust protective mask, a part of a fuel cell, glass, paper, wood, leather, fur, asbestos, brick, cement, a metal and a metal oxide, a ceramic product, plastic, a painted surface, and plaster.

3. The method according to claim 1 or 2, wherein an incident-angle variable ATR apparatus is used.

4. The method according to any one of claims 1 to 3, wherein the fluorine-containing polymer having a repeating unit derived from a fluorine-containing monomer containing the perfluoroalkyl group having 4 carbon atoms is used as a standard sample.

5. The method according to any one of claims 1 to 4, wherein
   Z is:

   a direct bond, a linear alkylene group or a branched alkylene group having 1 to 20 carbon atoms,
   or
   a group represented by the formula $-SO_2N(R^1)R^2-$ or the formula $-CON(R^1)R^2$ wherein $R^1$ is an alkyl group having 1 to 10 carbon atoms and $R^2$ is a linear alkylene group or branched alkylene group having 1 to 10 carbon atoms,
   or
   a group represented by the formula $-CH_2CH(OR^3)CH_2-$ wherein $R^3$ represents a hydrogen atom, or an acyl group having 1 to 10 carbon atoms,
   or
   a group represented by the formula $-Ar-CH_2-$ wherein Ar is an arylene group,
   or
   a $-(CH_2)_m-SO_2-(CH_2)_n-$ group or a $-(CH_2)_m-S-(CH_2)_n-$ group wherein m is 1 to 10 and n is 0 to 10.

6. The method according to any one of claims 1 to 5, wherein the article comprising the fluorine-containing surface treatment agent is analyzed without performing IR-ATR measurement of an article free of a fluorine-containing surface treatment agent.

Fig. 1

Fig. 2

(a)

1149cm⁻¹

(b)

1145cm⁻¹

Fig. 3

(a)

1149cm⁻¹

(b)

1144cm⁻¹

Fig. 4

## INTERNATIONAL SEARCH REPORT

International application No.

PCT/JP2016/073971

| A. CLASSIFICATION OF SUBJECT MATTER |
|---|
| *G01N21/35*(2014.01)i |

According to International Patent Classification (IPC) or to both national classification and IPC

| B. FIELDS SEARCHED |
|---|
| Minimum documentation searched (classification system followed by classification symbols) |
| G01N21/00, 21/01, 21/17-21/61, G01J3/00-3/52 |

Documentation searched other than minimum documentation to the extent that such documents are included in the fields searched

| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII)

| C. DOCUMENTS CONSIDERED TO BE RELEVANT | | |
|---|---|---|
| Category* | Citation of document, with indication, where appropriate, of the relevant passages | Relevant to claim No. |
| A | JP 2012-185045 A (Tokyo University of Science), 27 September 2012 (27.09.2012), paragraphs [0052], [0088] (Family: none) | 1-6 |
| A | JP 2013-217751 A (National University Corporation Chiba University), 24 October 2013 (24.10.2013), paragraph [0024] (Family: none) | 1-6 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 November 2016 (07.11.16) | 15 November 2016 (15.11.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

16

**INTERNATIONAL SEARCH REPORT**

| International application No. |
|---|
| PCT/JP2016/073971 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2005-522694 A  (Cascade Technologies Ltd.), 28 July 2005 (28.07.2005), paragraph [0037] & US 2005/0157303 A1 paragraph [0063] & GB 208100 D          & GB 208100 D0 & WO 2003/087787 A1     & EP 1493017 B1 & CA 2482402 A          & CN 1659429 A & RU 2004132718 A       & AU 2003219320 A & KR 10-0959625 B1      & ES 2392834 T | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015020100 A

### Non-patent literature cited in the description

- **K. HONDA ; M. MORITA ; H. OTSUKA ; A. TAKAHARA.** *Macromolecules,* 2005, vol. 38 (13), 5699-5705
- **K. HONDA ; M. MORITA ; O. SAKATA ; S. SASAKI ; A. TAKAHARA.** *Macromolecules,* 2010, vol. 43 (1), 454-460
- **TAKESHI HASEGAWA et al.** Stratified Dipole-Arrays Model Accounting for Bulk Properties Specific to Perfluoroalkyl Compounds. *ChemPlusChem,* 2014, vol. 79, 1421-1425
- **TAKESHI HASEGAWA et al.** An Origin of Complicated Infrared Spectra of Perfluoroalkyl Compounds Involving a Normal Alkyl Group. *Chemistry Letters,* 2015, vol. 44, 834-836
- **TAKESHI HASEGAWA.** Understanding of the intrinsic difference between normal- and perfluoro-alkyl compounds toward total understanding of material properties. *Chemical Physics Letters,* 2015, vol. 627, 64-66
- **K. HONDA et al.** *Macromolecules,* 2005, vol. 38 (13), 5699-5705
- **K. HONDA et al.** *Macromolecules,* 2010, vol. 43 (1), 454-460
- **T. HASEGAWA et al.** An Origin of Complicated Infrared Spectra of Perfluoroalkyl Compounds Involving a Normal Alkyl Group. *Chemistry Letters,* 2015, vol. 44, 834-836
- **T. HASEGAWA.** Understanding of the intrinsic difference between normal- and perfluoro-alkyl compounds toward total understanding of material properties. *Chemical Physics Letters,* 2015, vol. 627, 64-66
- **V.P. TOLSTOY ; I.V. CHEMYSHOWA ; V.A. SKRYSHEVSKY.** Handbook of Infrared Spectroscopy of Ultrathin Films. Wiley, 2003